# EUROPÄISCHE PATENTSCHRIFT

(11) **EP 1 115 480 B1**
(45) Veröffentlichungstag und Bekanntmachung des Hinweises auf die Patenterteilung: **06.10.2004**
(21) Anmeldenummer: 99932810.7
(22) Anmeldetag: 03.07.1999
(51) Int. Cl.: B01J 13/18

(54) **MIKROPARTIKEL HERGESTELLT AUS CYCLOOLEFINCOPOLYMEREN UND DEREN VERWENDUNG ZUR KONTROLLIERTEN WIRKSTOFFFREIGABE**
MICROPARTICLES PRODUCED FROM CYCLIC OLEFIN COPOLYMERS AND THEIR USE FOR THE CONTROLLED RELEASE OF ACTIVE AGENTS
MICROPARTICULES FABRIQUEES A PARTIR DE COPOLYMERES DE CYCLO-OLEFINES ET LEUR UTILISATION POUR LA LIBERATION CONTROLEE DE CONSTITUANTS ACTIFS

(30) Priorität: 28.07.1998 DE 19834025
(43) Veröffentlichungstag der Anmeldung: 18.07.2001
(73) Patentinhaber: Bayer CropScience GmbH, 65929 Frankfurt/Main (DE)
(72) Erfinder: RUEPPEL, Diether, D-65929 Frankfurt am Main (DE); HAHN, Bernd, D-65597 Hünfelden (DE); SCHNELLER, Arnold, D-64409 Messel (DE); BERGER, Klaus, D-65830 Kriftel (DE); OSAN, Frank, D-65779 Kelkheim (DE)
(86) Internationale Anmeldenummer: PCT/EP1999/004642
(87) Internationale Veröffentlichungsnummer: WO 2000/006296

(56) Entgegenhaltungen:
- EP-A- 0 754 562
- WO-A-97/48740
- US-A- 4 661 104
- PATENT ABSTRACTS OF JAPAN vol. 014, no. 533 (E-1005), 22. November 1990 (1990-11-22) & JP 02 224354 A (NEC CORP), 6. September 1990 (1990-09-06)
- PATENT ABSTRACTS OF JAPAN vol. 003, no. 157 (C-068), 22. Dezember 1979 (1979-12-22) & JP 54 132235 A (IDEMITSU KOSAN CO LTD), 15. Oktober 1979 (1979-10-15)

## Beschreibung

Die vorliegende Erfindung betrifft Mikropartikel aus Cycloolefincopolymeren, ein Verfahren zur Herstellung sowie deren Verwendung zur kontrollierten Freigabe von Wirkstoffen, vorzugsweise von Agrochemikalien, gegebenenfalls unter Verwendung von Formulier- und Hilfsstoffen, vorzugsweise Diatomeenerde.

In der modernen agrarchemischen Technologie werden Formulierungen und Wirkstoffkombinationen immer bedeutender, deren Anwendungsform Einfluß auf die Bioverteilung und Bioverfügbarkeit nehmen.

Mikropartikel finden vor allem auf dem Gebiet von Depotformulierungen Anwendung, wobei der in den Mikropartikel enthaltene Wirkstoff aufgrund der Mikropartikelhülle oder -matrix, statt sofort, verzögert freigesetzt wird - sogenannte kontrollierte Wirkstofffreigabe (controlled release). Insbesondere Mikropartikel, die eine Teilchengröße im Bereich zwischen 1 -1000 µm aufweisen, erweisen sich als aussichtsreiche Formulierungen.

Die kontrollierte Freigabe insbesondere von Agrochemikalien über einen längeren Zeitraum hat mehrere Vorteile. Zum einen kann das mehrfache Ausbringen der Agrochemikalien auf eine einmalige Applikation reduziert werden. Zum anderen werden lokale Über- und Unterdosierungen vermieden. Ein schnelles Ausschwemmen oder der Abbau der Wirkstoffe können verhindert werden.

Im Stand der Technik werden Mikrosysteme aus verschiedenen Materialien und mit verschiedenen Geometrien beschrieben. Entsprechend unterschiedlich sind die Herstellungsprozeduren. Bekannt sind Mikrokapseln aus Polyethylen oder Ethylen-Copolymeren. Ethylen-Cycloolefin Copolymere werden zwar im Zusammenhang mit Mikrokugeln erwähnt, nicht jedoch für die Wirkstofffreigabe sondern für Kleber und Pulverlacke (WO 97/48740 A). In JP 05080232 B werden reine Polynorbornene zur Freigabe eines Parfums verwendet. Von Nachteil sind die hohen Verarbeitungstemperaturen der Polynorbornen-Homopolymeren.

Im Stand der Technik sind Druckschriften gegeben, die sich mit der Freigabe von Wirkstoffen aus Ethylen-(co)- polymeren befassen. In US 4,002,458 A werden Kapseln mit Kern-Schale Geometrie beschrieben. Polyethylenhüllen werden mittels einer Düse aufgebracht. Es entstehen bis zu 2 Millimeter große Kapseln. Im Gegensatz zur Kem-Schale Geometrie ist bei der vorliegenden Erfindung der Wirkstoff in einer Polymermatrix eingebettet. Ethylen -Propylen-Copolymere werden in US 4,405,360 A verwendet, nicht jedoch als Partikel sondern als plattenförmiger Spender. In US 4,299,613 A werden Formkörper aus Ethylen-Vinylacetat Copolymere hergestellt; Mikropartikel werden jedoch nicht erwähnt. EP 529975 A beschreibt die Verwendung von Ethylen-Vinylacetat Copolymeren in Form von Granulaten. Häufiger werden auch Polyethylenglycole genannt (US 5,441,923), die jedoch wasserlöslich sind.

JP54- 132235A offenbart Mikropartikeln, von denen sich der Gegenstand des Anspruchs 1 dadurch unterscheidet, daß die Auswahl eines Cycloolefincopolymers als Matrixmaterial nicht offenbart wird. Die Partikeln bestehen aus eine Mischung von niedermolekularen und organischen Feststoffen, welche mittels Schmelze zusammengeführt werden. Der Wirkstoff ist eine Agrochemikalie, die kontrolliert freigegeben wird.

Mikropartikel zur kontrollierten Wirkstofffreigabe aus Cycloolefincopolymeren sind im Stand der Technik nicht beschrieben und stellen in diesem Zusammenhang ein neuartiges Matrixmaterial dar.

Als vorteilhaft wird angesehen:
- Hervorragende Biokompatibilität und hohe Reinheit der Polymere als Matrixmaterial, so daß diese Werkstoffe ohne Gefährdung für Flora und Fauna als Basismaterialien für Mikropartikel einsetzbar sind.
- Der geringe Gehalt an witterungsempfindlichen Doppelbindungen bewirkt eine hohe Lagerstabiliät der erfindungsgemäßen Mikropartikel.
- Die hohe Fließfähigkeit des Basismaterials sorgt für eine erleichterte Verarbeitbarkeit.
- Die Dimensionsstabilität, mechanische Festigkeit, Steifheit und Härte der Matrixmaterialien führt zu verbesserter Handhabbarkeit der erfndungsgemäßen Mikropartikel.
- Hohe Beständigkeit der Mikropartikel gegenüber Säuren, Laugen und polaren oder mäßig polaren Medien bedeuten Vorteile bei der Lagerung und bei der Handhabung.
- Die geringe Dichte des Matrixmaterials bietet Vorteile bei Transport, Lagerung und Anwendung.
- Durch die abgestufte Wärmeformbeständigkeit, die in weiten Bereichen variable Molmasse und den veränderbaren Kristallisationsgrad von Cycloolefincopolymeren kann das Eigenschaftsprofil des Matrixmaterials auf die jeweilige Anwendung hin abgestimmt werden.

Überraschenderweise zeigen Experimente, daß vorzugsweise Formulier- und Hilfsstoffe die gewünschte kontrollierte Freigabe von Wirkstoffen aus dem vorteilhaft beschriebenem Matrixmaterial bewirken.

Dies ist besonders überraschend, da die Matrixmaterialien der erfindungsgemäßen Mikropartikel als Konstruktionswerkstoffe eingesetzt werden (z.B. Topas®).

Die Aufgabe der vorliegenden Erfindung besteht daher darin, Mikropartikel aus Cycloolefincopolymeren, vorzugsweise Ethylen-Norbornen-Copolymeren, als Wirkstoffträger zur kontrollierten Freigabe von Wirkstoffen, gegebenenfalls unter Verwendung geeigneter Formulier- und Hilfsstoffe, bereitzustellen.

Die Aufgabe wird dadurch gelöst, daß Mikropartikel aus thermoplastischen Cycloolefincopolymeren, vorzugsweise Ethylen-Norbornen-Copolymeren und Wirkstoffen erhalten werden, welche gegebenenfalls unter der Verwendung von Formulier- und Hilfsstoffen, vorzugsweise Diatomeenerde, eine kontrollierte Freigabe der Wirkstoffe ermöglichen.

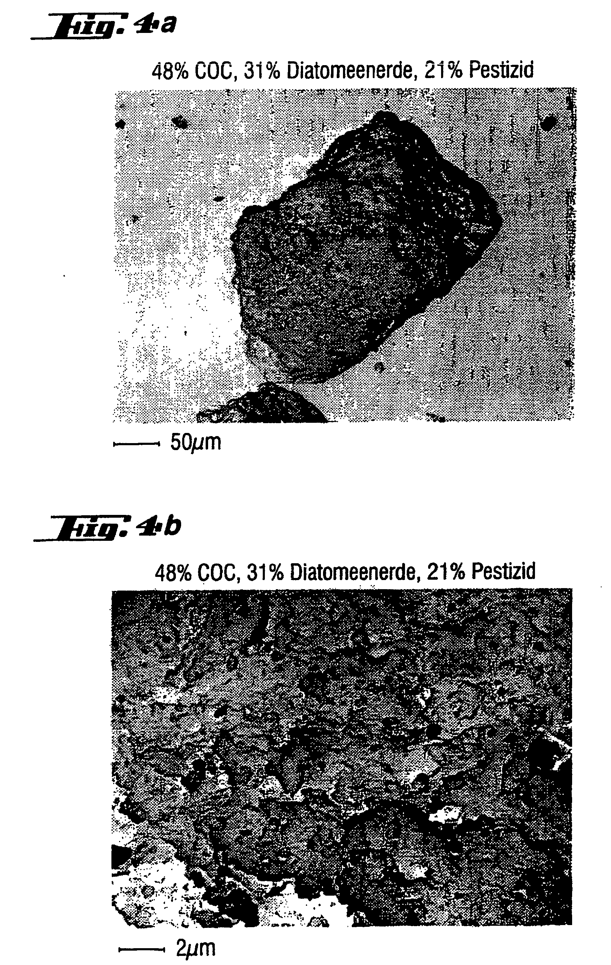

Ein Gegenstand der Erfindung sind daher Mikropartikel erhältlich aus thermoplastischen Cycloolefincopolymeren (im folgenden "COC") und Wirkstoffen. Die Wirkstoffe sind in einer Polymermatrix aus mindestens einem thermoplastischen Cycloolefincopolymer, vorzugsweise Ethylen-Norbornen-Copolymeren, eingebettet und bilden ein Konglomerat (Figur 4).

Im Sinne dieser Erfindung bedeutet Mikropartikel daher ein Formgebilde aus Matrixmaterialien obiger Polymere mit einem mittleren Durchmesser von 1 bis 1000 µm vorzugsweise 10-900 µm, besonders bevorzugt 50-800 µm und ganz besonders bevorzugt 100 - 600 µm. Nach- und vorstehend als erfindungsgemäße Mikropartikel bezeichnet.

Ein weiterer Gegenstand der Erfindung ist die Verwendung der erfindungsgemäßen Mikropartikel als Wirkstoffträger zur kontrollierten Freigabe von Wirkstoffen. Hierzu können in die erfindungsgemäßen Mikropartikel Formulier- und Hilfsstoffe neben den Wirkstoffen eingetragen, die eine kontrollierte Wirkstofffreigabe ermöglichen. Als besonders bevorzugt ist Diatomeenerde und/oder Kieselalge anzusehen. Entsprechend können ebenfalls Kieselgel oder dem Fachmann bekanntes entsprechendes Material verwendet werden. Denkbar sind auch anorganische Stoffe entsprechender Polarität und/oder Amorphität. solche Stoffe sind ausdrücklich umfaßt. Zudem können die erwähnten Formulier- und Hilfsstoffe in Kombination mit bekannten Formulier- und Hilfsstoffen verwendet werden, wie Cellulose, Salze etc.

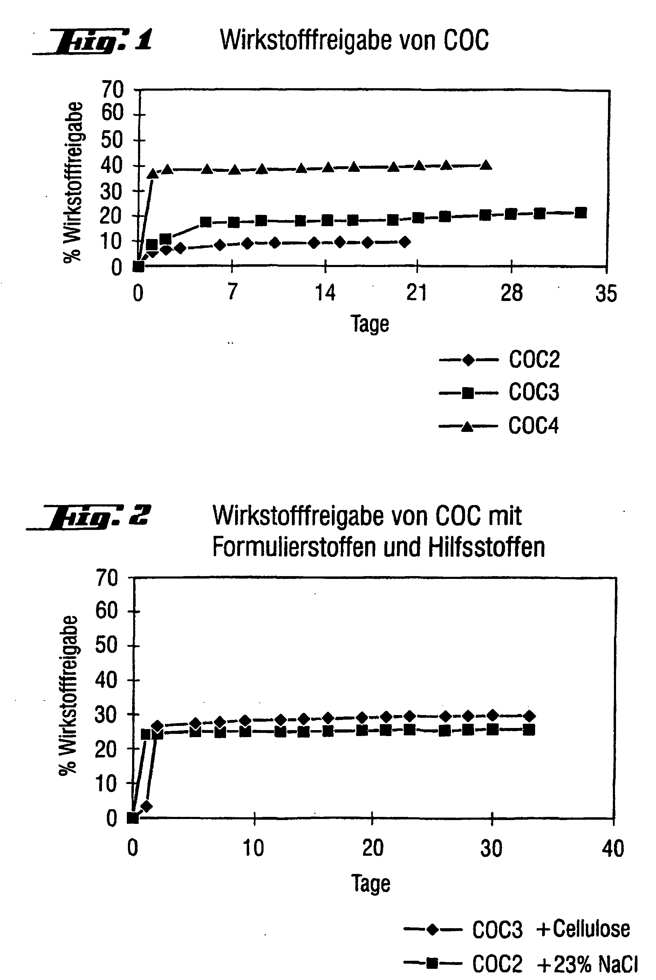

Prinzipiell kann auch ohne ein Formulier- und Hilfsstoff eine kontrollierte Wirkstoffreigabe erhalten werden, wie aus Fig. 1 ersichtlich. Dies ist insbesondere bei Eingabe von hydrophoben Wirkstoffen in die erfindungsgemäße Mikropartikel zu erwarten.

Die kontrollierte Freigabe hydrophiler Wirkstoffe kann in einem breiten Rahmen erfolgen durch Auswahl und Kombination der Matrixmaterialien aus Cycloolefincopolymeren und zusätzlicher Verwendung von Formulier- oder Hilfsstoffe.

Als Wirkstoff wird im Rahmen dieser Erfindung jede biologisch aktive Substanz und Substanzkombination im weitesten Sinne angesehen, vorzugsweise pharmazeutische Wirkstoffe, besonders bevorzugt jedoch Agrochemikalien, die in Landwirtschaft und Gartenbau eingesetzt werden können.

Unter dem Begriff Agrochemikalie fallen Düngemittel, Herbizide, Fungizide, Insektizide und andere Pflanzenschutz- und Schädlingsbekämpfungsmittel, Vorratsschutzmittel, Pflanzenwuchs- und -hemmstoffe, Silierungs-, Konservierungsmittel sowie Bodenverbesserungsmittel. Selbst Futtermittelzusätze, Tierhygiene- und -arzneimittel oder Aroma- und Duftstoffe werden hierbei nicht ausgeschlossen.

Beispielsweise sind bekannte Wirkstoffe einsetzbar, wie sie z.B. aus Weed Research 26, 441-445 (1986) oder "The Pesticide Manual", 11th edition, The British Crop Protection Council and the Royal Soc. of Chemistry, 1997 und dort zitierter Literatur beschrieben sind. Als bekannte Herbizide, die in den erfindungsgemäßen Wirkstoffträger eingetragen werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor, acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor, alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h.
5-Fluor-2-phenyl-4H-3,1-benzoxazin-4-on; benazolin; benfluralin; benfuresate; bensulfuron-methyl; bensulide; bentazone; benzofenap; benzofluor; benzoylpropethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h.
Diethyldithiocarbaminsäure-2-chlorallylester, chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole;
daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr, diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuronmethyl; ethidimuron; ethiozin; ethofumesate; F5231, d.h.
N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester, haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor, methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h.
6-Chlor-N-(3-chlor-2-propenyl)-5-methyl-N-phenyl-3-pyridazinamin; MT-5950, d.h. N-[3-Chlor-4-(1-methylethyl)-phenyl]-2-methylpentanamid-, napropanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor, propanil; propaquizafop und dessen Ester; propazine; propham; propisochlor, propyzamide; prosulfalin; prosulfocarb; prosulfuron (CGA-152005); prynachlor, pyrazolinate; pyrazon; pyrazosulfuron-ethyl; pyrazoxyfen; pyridate; pyrithiobac (KlH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor, terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085);
thifensulfuron-methyl; thiobencarb; tiocarbazil; tralkoxydim; tri-allate; triasulfuron; triazofenamide; tribenuron-methyl; triclopyr, tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Ein weiterer Gegenstand der Erfindung betrifft eine agrochemische Zusammensetzung der erfindungsgemäßen Mikropartikel. Hierbei wird eine oder mehrere Agrochemiekalien gegebenenfalls mit Formulier- und Hilfsstoffen, vorzugsweise Diatomeenerde in die erfindungsgemäße Mikropartikel eingebracht und formuliert und dient vorzugsweise zur Ausbringung in der Landwirtschaft.

Ein weiterer Gegenstand der Erfindung betrifft eine pharmazeutische Zusammensetzung der erfindungsgemäßen Mikropartikel. Hierbei wird ein oder mehrere pharmazeutische Wirkstoffe gegebenenfalls mit Formulier- und Hilfsstoffen, vorzugsweise Diatomeenerde in die erfindungsgemäße Mikropartikel eingebracht und formuliert.

Unter einer "kontrollierten Wirkstoffabgabe oder kontrollierten Wirkstofffreigabe" wird verstanden, daß die Wirkstoffe unter Akzeptanz einer den Umständen. entsprechenden statistischen Abweichung nach einer bestimmten Zeit und/oder Zeitdauer in einer für den biologischen Organismus vorteilhaften Dosis freigesetzt werden.
Diese Definition beinhaltet auch Extreme. Zum einen die spontane Freigabe der in der Formulierung vorliegenden Wirkstoffe innerhalb einer auf den Wert Null zugehenden Zeitdauer. Zum anderen die minimal erforderliche Menge/Dosis zur Erzielung eines Effekts über eine lange Zeitdauer bis sämtliche in der Formulierung vorliegenden Wirkstoffe freigesetzt sind.

Daher wird für die hier vorliegende Formulierung synonym von einer Depotformulierung oder Formulierung mit kontrollierter Freisetzung gesprochen.

Darüber hinaus zeigt die vorliegende Erfindung, daß eine weitere Veränderung des Freisetzungsprofils durch Auswahl geeigneter Matrixmaterialien (Beispiele 6,7,8 zur Figur 1) und/oder Zusatz polarer Formulier- und Hilfsstoffe möglich ist. Durch Zugabe von Kochsalz oder Zellulose kann die anfängliche Wirkstofffreigabe erhöht werden (Beispiel 9,10 zur Figur 2). Durch die Zugabe von Diatomeenerde wird überraschenderweise ein völlig anderes Freigabeprofil erhalten: Es wird deutlich mehr Wirkstoff über einen längeren Zeitraum abgegeben. Weitere Erhöhung der Wirkstoffabgabe erreicht man durch Verwendung spezifischer "COC"-Typen (Beispiel 11,12,13 zur Figur 3).

In einer bevorzugten Ausführungsform werden daher Matrixmaterialien für die erfindungsgemäßen Mikropartikel eingesetzt, welche vorzugsweise mindestens ein Cycloolefincopolymer, ausgewählt aus Polymeren, enthaltend 0,1 bis 99,9 Gew.-% (bezogen auf die Gesamtmasse des Cycloolefincopolymeres) polymerisierte Einheiten mindestens eines cyclischen Olefins und 0,1 bis 99,9 Gew.-% (bezogen auf die Gesamtmasse des Cycloolefincopolymers) polymerisierte Einheiten eines acyclischen Olefins enthalten.

Besonders bevorzugt enthalten die Matrixmaterialien der erfindungsgemäßen Mikropartikel Olefine mit Norbornengrundstruktur, ganz besonders bevorzugt Norbornen und Tetracyclododecen. Bevorzugt sind auch Cycloolefincopolymere, die polymerisierte Einheiten enthalten, die sich ableiten von acyclischen Olefinen mit endständigen Doppelbindungen wie alpha-Olefinen mit 2 bis 20 C-Atomen, besonders bevorzugt sind Ethylen oder Propylen. Ganz besonders bevorzugt sind Norbornen-Ethylen- und Tetracyclododecen-Ethylen-Copolymere.

Zur Ausführung der Erfindung werden die Matrixmaterialien der erfindungsgemäßen Mikropartikel durch eine heterogene oder homogene Katalyse mit metallorganischen Verbindungen hergestellt. Verwendbar sind Katalysatorsysteme basierend auf Mischkatalysatoren aus Titansalzen und Aluminiumorganylen, wie in DD-A-109 224 und DD-A-237 070 beschreiben. EP-A-156 464, EP 0 582 355 und EP 0 466 279 beschreiben die Herstellung mit Katalysatoren auf Vanadiumbasis. EP-A-283 164, EP-A-407 870, EP-A-485 893 und EP-A-503 422 beschreiben deren Herstellung mit Katalysatoren basierend auf löslichen Metallocenkomplexen. Auf die in diesen Patenten zur Herstellung von Cycloolefincopolymeren beschriebenen Herstellungsverfahren und verwendeten Katalysatorsysteme wird ausdrücklich Bezug genommen.

Die können ebenfalls mittels ringöffnende Polymerisation von Cycloolefinen und anschließende Hydrierung der so erhaltenen Produkte gemäß den japanischen Patenten JP 3-14882, JP 3-122137, JP 4-63807, JP 2-227424 und JP 2-276842 hergestellt werden. Eingeschlossen sind ebenfalls Derivate dieser cyclischen Olefine mit polaren Gruppen, wie Halogen-, Hydroxyl-, Ester-, Alkoxy-, Carboxy-, Cyano-, Amido-, Imido- oder Silylgruppen.

Für die erfindungsgemäßen Mikropartikel sind als Matrixmaterialien ebenfalls Mischungen aus Cyccloolefincopolymeren und Polyolefinen geeignet. Hierfür können bevorzugt folgende Polyolefine eingesetzt werden: Homopolymere des Ethylens und Propylens und Copolymere davon; Copolymere auf der Basis von Ethylen mit linearen oder verzweigten Olefinen, wie Buten, Penten, Hexen, Hepten, Octen, Nonen, Decen, Undecen und Dodecen, Copolymere auf der Basis von Propylen mit linearen oder verzweigten Olefinen, wie Buten, Penten, Hexen, Hepten, Octen, Nonen, Decen, Undecen und Dodecen, Terpolymere aus Ethylen, Propylen und linearen oder verzweigten Olefinen, wie Buten, Penten, Hexen, Hepten, Octen, Nonen, Decen, Undecen und Dodecen. "COC" auf der Basis von Comonomeren, wie Ethylen und 2-Norbornen sind amorphe oder teilkristalline, transparente Werkstoffe. Die Wärmeformbeständigkeiten der Cycloolefincopolymere lassen sich durch die Variation der Anteile der Comonomere in einem weiten Bereich einstellen. Als Anhaltspunkt für die Wärmeformbeständigkeit, wie sie nach ISO 75 Teil 1 und Teil 2 (entspricht DIN 53461, Deutsches Institut für Normung, Berlin, 9. Auflage, 1988, S. 198) an Spritzgußformkörpem bestimmt werden kann, läßt sich für Cycloolefincopolymere die Glasübergangstemperatur heranziehen. Die beschriebenen Cycloolefincopolymere weisen Glastemperaturen zwischen -20 und 220°C auf.

Die mittlere Molmasse der "COC" läßt sich durch Wasserstoff-Dosierung, Variation der Katalysatorkonzentration oder Variation der Temperatur in bekannter Weise steuern. Die "COC" weisen massenmittlere Molmassen Mw zwischen 1.000 und 10.000.000 g/mol auf. Bevorzugt sind massenmittlere Molmassen Mw zwischen 1.000 und 5.000.000 g/mol, besonders bevorzugt sind massenmittlere Molmassen Mw zwischen 1.000 und 1.200.000 g/mol. Die in den erfindungsgemäßen Matrixmaterialien für Mikropartikel enthaltenen Cycloolefincopolymere weisen Viskositätszahlen (VZ) zwischen 5 und 1.000 ml/g auf. Bevorzugt sind Viskositätszahlen zwischen 5 und 500 ml/g, besonders bevorzugt sind Viskositätszahlen zwischen 5 und 300 ml/g.

Die Matrixmaterialien der erfindungsgemäßen Mikropartikel sind thermoplastische Materialien. Daher lassen sie sich mit allen bekannten Verfahren zur Verarbeitung von thermoplastischen Polymeren verarbeiten. Dazu zählen unter anderem Extrudieren von Folien und Fasern, Extrusionsblasformen von Folien und Flaschen, Spritzblasformen, Spritzgießen und Kalandrieren. Die Fließfähigkeiten der Schmelzen lassen sich über die Variation der Glasstufen und der Molekulargewichte einstellen und an die Bedingungen der Verarbeitungsmethode anpassen.

"COC" lassen sich auch aus der Lösung verarbeiten. Geeignete Lösungsmittel sind aprotische unpolare Kohlenwasserstoffe wie Dekalin oder Gemische aus linearen und verzweigten Kohlenwasserstoffen.

Sowohl beim Extrudieren als auch beim Spritzgießen wurden bei Temperaturen von 300°C weder Zersetzungsreaktionen noch ein Viskositätsabbau gefunden.

Die Eigenschaften der Matrixmaterialien der erfindungsgemäßen Mikropartikel können sowohl intrinsisch wie auch durch die Zugabe von Hilfs- und Zusatzstoffen, wie z.B. Weichmachern verändert werden. Optimierte Mischungen können beispielsweise Wachse, Öle, Tenside, Emulgatoren, Fette, Fasern, Füllstoffe und Verstärkungsmittel, Aktivkohle, poröse Stoffe, Salze, allgemein polare Stoffe, Silikate, Zeolithe, Weichmacher, Antioxidantien, UV-Absorber und Lichtschutzmittel, Acrylate, Nickelverbindungen, sterisch gehinderte Amine, Oxalsäurediamide, Phosphite und Phosphonite, peroxidzerstörende Verbindungen, basische Costabilisatoren, Nukleierungsmittel, Gleitmittel, Pigmente, Farbmittel, Flammschutzmittel, Antistatika, Biostabilisatoren, optische Aufheller, Treibmittel, organische Peroxide, sowie weitere typische Kunststoffadditive und Verarbeitungshilfsmittel enthalten.

Zur weiteren Ausführung der Erfindung werden verschiedene Ethylen-Norbornen Copolymere zusammen mit dem Wirkstoff und Füllstoffen wie Kochsalz, Zellulose oder Diatomeenerde durch Kneten vermischt. Die Temperaturen liegen bei 100°C. Die Masse wird anschließend gemahlen. Die Teilchengröße liegt zwischen 100 µm - 1000 µm. Der Anteil der Formulier- ,Hilfs- und Zusatzstoffe kann zwischen 5 und 50% liegen. Dadurch kann das Freigabeverhalten in der Regel zu höheren Freigaberaten beeinflußt werden. Die Konzentration des Wirkstoffes kann zwischen 1- 50% liegen. Als Pflanzenschutzmittel wird Ethoxysulfuron (3-(4,6-dimethoxypyrimidin-2-yl)-1-(2-ethoxyphenoxysulfonyl)-urea) verwendet, ohne daß die Erfindung darauf beschränkt ist. Die Freigabe des Wirkstoffes kann in vitro gemessen werden. Die Freigabezeiten liegen im Bereich von 10 Tagen bis ca. 4 Wochen.
Figur 1 zeigt die Wirkstofffreigabe der erfindungsgemäßen Mikropartikel gemäß Beispiel 6,7 und 8.
Figur 2 zeigt die Wirkstofffreigabe der erfindungsgemäßen Mikropartikel gemäß Beispiel 9 und 10.

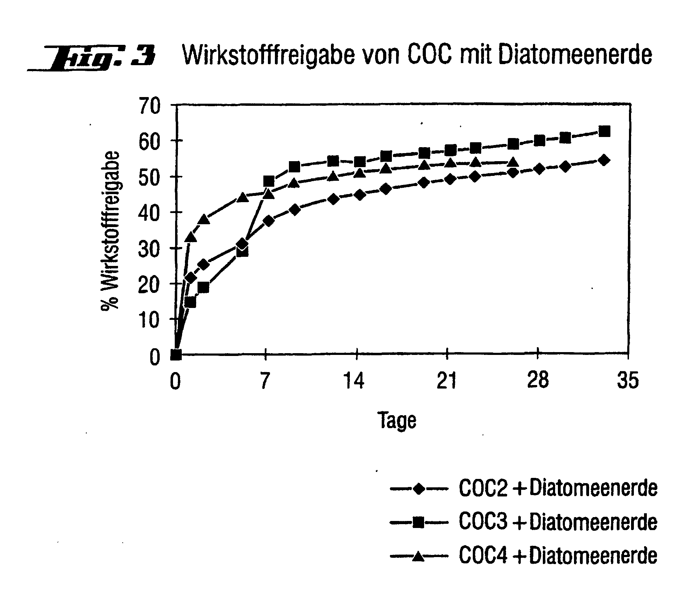

Figur 3 zeigt die Wirkstofffreigabe der erfindungsgemäßen Mikropartikel gemäß Beispiel 11, 12 und 13.
Figur 4 zeigt rasterlelektronische Aufnahmen der Mikropartikel in (a) 200 -facher und (b) 5000 -facher Vergrößerung.

Die nachfolgenden Beispiele dienen zur näheren Erläuterungen der Erfindung, ohne dieselbe auf in den Beispielen beschriebenen Produkte und Ausführungsformen einzuschränken.

### Beispiele

"COC", die als Matrixmaterial in der erfindungsgemäßen Mikropartikel dienen.

### Beispiel 1

In einem 70dm³-Autoklav, der vorher mit Ethen gespült wurde, wurde eine 48 Gew.-%ige Lösung von Norbornen in Toluol vorgelegt. Durch mehrfaches Aufdrücken von Ethen wurde die Lösung mit Ethen gesättigt. Dem auf diese Weise vorbereitete Reaktor wurde im Gegenstrom einer toluolhaltigen Lösung von Methylaluminiumoxanlösung (10 Gew.-%ige Methylaluminiumoxanlösung der Molmasse 1300g/mol nach kryoskopischer Bestimmung) dosiert und 30 Minuten bei 70°C gerührt. Eine Lösung von insgesamt 30 mg des Metallocens Isopropylen-bis-(1-indenyl)-zirkondichlorid in toluolhaltiger Lösung wurde nach 30 minütiger Voraktivierung zugegeben.

Unter Rühren wurde eine Stunde polymerisiert, wobei der Ethylendruck von 20 bar durch Nachdosieren konstant gehalten wurde. Die Menge an Wasserstoff betrug 2000ppm.

Nach Ende der Reaktionszeit wurde das Polymerisationsgemisch in ein Gefäß abgelassen und sofort in 300dm³ Aceton eingetragen, 30 Minuten gerührt und das ausgefallenen Produkt anschließend filtriert. Der Filterkuchen wurde je dreimal abwechselnd mit 10%iger Salzsäure und Aceton gewaschen; der Rückstand in Aceton aufgeschlämmt und erneut filtriert. Das gereinigte Produkt wurde bei 40°C im Vakuum (0,2bar) 24 Stunden getrocknet.
Es wurde ein farbloses Polymer mit einer VZ von 92ml/g, einer Glasumwandlungstemperatur von 80°C und einer massenmittleren Molmasse Mw=52300g/mol erhalten.

Dies Produkt wird im folgenden als "COC 1" bezeichnet.

### Beispiel 2

Die anderen Produkte wurde durch Variation der Art des eingesetzten Metallocens, des Wasserstoffdruckes und der Norbomenmenge hergestellt.
Als "COC 2" wird ein Produkt mit einer VZ von 15ml/g einer Glasumwandlungstemperatur von 55°C und einer massenmitleren Molmasse von 6400g/mol bezeichnet.

### Beispiel 3

"COC 3" ist ein teilkristalines Cycloolefincopolymer mit einer VZ von 70ml/g, einer Glasumwandlungstemperatur von -6°C, einem Schmelzpunkt von 69°C und einer massenmitleren Molmasse von 34000 g/mol.

### Beispiel 4

37,5g "COC 1" und 12,5 g des Weißöles Ondina G 41 (Shell) wurden in die auf 100°C erwärmt Mischkammer eines Meßkneters Rheomix 600 / Rheocord 90 von Haake eingewogen. Die Probe wurde anschließend so lange (etwa 30 Minuten) geknetet, bis das Drehmoment als Funktion der Zeit konstant blieb. Das erhaltenen homogene Produkt besaß eine Glasumwandlungstemperatur von 15°C. Im folgenden wird diese Mischung als "COC 4" bezeichnet.

Im folgenden wird die Herstellung von Mikropartikel aus "COC" gemäß Beispiel 1-4 beschrieben.

### Beispiel 5

Zur Herstellung der Mikropartikel aus Cycloolefincopolymeren als Matrixmaterial wurde ein Meßkneter Rheomix 600 / Rheocord 90 von Haake eingesetzt. Die Ausgangskomponenten werden unter Stickstoffüberlagerung in die auf 100°C erwärmte Mischkammer des Kneters eingefüllt. Anschließend wird die Probe bei dieser Temperatur über 15 Minuten mit 20 Umdrehungen pro Minute geknetet, so daß eine homogene Mischung entsteht. Die homogene Verteilung der Komponenten kann daran erkannt werden, daß das Drehmoment als Funktion der Zeit konstant bleibt

Das Produkt wird anschließend aus dem Kneter entfernt, mit einer Analysenmühle gemahlen und mit Hilfe der Siebanalyse in verschiedene Größenfraktionen aufgetrennt.

Zur Herstellung größerer Mengen werden die Ausgangskomponenten in den Einfüllstutzen eines Extruders gefüllt (Leistritz GL34, Doppelschneckenextruder). Der Extruder wird bei 100°C mit 100 Umdrehungen /min betrieben. Die Ausbeute beträgt ca. 4 kg/h. Vorteilhaft ist, daß das Extrudat sehr schnell erstarrt, so daß es schnell gebrochen werden kann. Das Produkt wird anschließend gemahlen und durch Sieben in die gewünschten Fraktionen getrennt.

Beispiele 6 - 13 für die Art und Menge der verwendeten Ausgangskomponenten:
6. "COC 2": 3,43g Wirkstoff "Ethoxysulfuron"
7. "COC 3": 3,43g Wirkstoff "Ethoxysulfuron"
8. "COC 4": 3,43g Wirkstoff "Ethoxysulfuron"
9. "COC 2": 21,6g NaCl, 3,23g Wirkstoff "Ethoxysulfuron"
10. "COC 3": 22,3g Cellulosefasern FIC 200, 3,43g Wirkstoff Ethoxysulfuron
11."COC 2": 22,3g Diatomeenerde, 3,43g Wirkstoff Ethoxysulfuron
12."COC 3": 22,3g Diatomeenerde, 3,43g Wirkstoff Ethoxysulfuron
13."COC 4": 22,3g Diaomeenerde, 3,43g Wirkstoff Ethoxysulfuron

Die Freigabe des Wirkstoffes wird über die UV-Absorption gemessen. Siehe Beispiele in den Figuren 1-3.

## Patentansprüche

1. Mikropartikel zur kontrollierten Wirkstofffreigabe, enthaltend mindestens ein thermoplastisches Cycloolefincopolymer und einen oder mehrere Wirkstoffe.

2. Mikropartikel nach Anspruch 1, **dadurch gekennzeichnet, daß** das Cycloolefinpolymer ein Norbornen-Ethylen-Copolymer und/oder Tetracyclododecen-Ethylen-Copolymer ist.

3. Mikropartikel nach Anspruch 1 und 2, **dadurch gekennzeichnet, daß** die Wirkstoffe in einer Matrix eingebettet sind.

4. Mikropartikel nach Anspruch 1 bis 3 mit einem mittleren Durchmesser von 1 - 1000 µm, vorzugsweise 100-600 µm.

5. Mikropartikel nach Anspruch 1 bis 4 enthaltend mindestens ein Formulier- und Hilfsstoff.

6. Mikropartikel nach Anspruch 5, **dadurch gekennzeichnet, daß** Diatomeenerde als Formulierstoff verwendet wird.

7. Mikropartikel nach einem oder mehreren der Ansprüche 1 bis 6, **dadurch gekennzeichnet, daß** dieser zusätzlich einen oder mehrere Wirkstoffe aus der Gruppe der Agrochemikalien oder Pharmaka enthält.

8. Mikropartikel nach einem der Ansprüche 1 bis 7 erhältlich durch Kneten und/oder Extrudieren und Mahlen.

9. Mikropartikel nach einem der Ansprüche 1-8, **dadurch gekennzeichnet, daß** die massenmittlere Molmasse des Cycloolefincopolymers 1 -10.000 kg/mol, vorzugsweise 1 bis 1200 kg/mol, beträgt.

10. Mikropartikel nach einem der Ansprüche 1-9, **dadurch gekennzeichnet, daß** die Viskositätszahl des Cycloolefincopolymers 5 - 1000 ml/g, vorzugsweise 5 - 300 ml/g, beträgt.

11. Mikropartikel nach einem der Ansprüche 1-10, **dadurch gekennzeichnet, daß** die Glasübergangstemperatur des Cycloolefincopolymers -20 bis 220 o Celsius beträgt.

12. Verwendung der Mikropartikel nach einem oder mehreren der Ansprüche 1 bis 11 zur kontrollierten Freigabe von Wirkstoffen.

13. Verwendung der Mikropartikel nach einem oder mehreren der Ansprüche 1 bis 11 zur kontrollierten Freigabe von Agrochemikalien.

14. Verwendung der Mikropartikel nach einem oder mehreren der Ansprüche 1 bis 11 als pharmazeutische Zusammensetzung.

15. Verwendung der Mikropartikel nach einem oder mehreren der Ansprüche 1 bis 11 als agrochemische Zusammensetzung.

## Claims

1. A microparticle for controlled active-substance release comprising at least one thermoplastic cycloolefin copolymer and one or more active substances.

2. A microparticle as claimed in claim 1, wherein the cycloolefin polymer is a norbornene-ethylene copolymer and/or tetracyclododecene-ethylene copolymer.

3. A microparticle as claimed un claim 1 or 2, wherein the active substances have been embedded in a matrix.

4. A microparticle as claimed in any of claims 1 to 3 with an average diameter of from 1 to 1000 µm, preferably from 100-600 µm.

5. A microparticle as claimed in any of claims 1 to 4 comprising at least one formulation auxiliary or other auxiliary.

6. A microparticle as claimed in claim 5, wherein the formulating auxiliary used comprises diatomaceous earth.

7. A microparticle as claimed in one or more of claims 1 to 6, which additionally comprises one or more active substances from the group comprising agrochemicals and pharmaceutical substances.

8. A microparticle as claimed in any one of claims 1 to 7, obtainable by kneading and/or extruding an grinding.

9. A microparticle as claimed in any one of claims 1 to 8, wherein the weight-average molar mass of the cycloolefin copolymer is from 1 to 10,000 kg/mol, preferably from 1 to 1200 kg/mol.

10. A microparticle as claimed in any one of claims 1 to 9, wherein the viscosity number of the cycloolefin copolymer is from 5 to 1000 ml/g, preferably from 5 to 300 ml/g.

11. A microparticle as claimed in any one of claims 1 to 10, wherein the glass transition temperature of the cycloolefin copolymer is from -20 to 220°C.

12. The use of the microparticles as claimed in one or more of claims 1 to 11 for the controlled release of active substances.

13. The use of the microparticles as claimed in one or more of claims 1 to 11 for the controlled release of agrochemicals.

14. The use of the microparticles as claimed in one or more of claims 1 to 11 as a pharmaceutical formulation.

15. The use of the microparticles as claimed in one or more of claims 1 to 11 as an agrochemical formulation.

## Revendications

1. Microparticule pour la libération contrôlée de substances actives, contenant au moins un copolymère de cyclooléfine thermoplastique et une ou plusieurs substances actives.

2. Microparticule selon la revendication 1, **caractérisée en ce que** le copolymère de cyclooléfine est un copolymère de norbornène-éthylène et/ou un copolymère de tétracyclododécène-éthylène.

3. Microparticule selon la revendication 1 ou 2, **caractérisée en ce que** les substances actives sont noyées dans une matrice.

4. Microparticule selon la revendication 1 à 3, avec un diamètre moyen de 1 à 1000 µm, de préférence de 100 à 600 µm.

5. Microparticule selon les revendications 1 à 4, contenant au moins un agent auxiliaire et de formulation.

6. Microparticule selon la revendication 5, **caractérisée en ce que** l'on utilise de la terre de diatomées en tant qu'agent de formulation.

7. Microparticule selon l'une ou plusieurs des revendications 1 à 6, **caractérisée en ce que** celle-ci contient de plus une ou plusieurs substances actives appartenant au groupe des produits agrochimiques ou pharmaceutiques.

8. Microparticule selon l'une des revendications 1 à 7, pouvant être obtenue par malaxage et/ou extrusion et broyage.

9. Microparticule selon l'une des revendications 1 à 8, **caractérisée en ce que** la masse moléculaire moyenne en masse du copolymère de cyclooléfine s'élève à 1-10 000 kg/mole, de préférence à 1-1200 kg/mole.

10. Microparticule selon l'une des revendications 1 à 9, **caractérisée en ce que** l'indice de viscosité du polymère de cyclooléfine est de 5 à 1000 ml/g, de préférence de 5 à 300 ml/g.

11. Microparticule selon l'une des revendications 1 à 10, **caractérisée en ce que** la température de transition vitreuse du copolymère de cyclooléfine s'élève de -20 à 220°C.

12. Utilisation de la microparticule selon l'une ou plusieurs des revendications 1 à 11, pour la libération contrôlée de substances actives.

13. Utilisation de la microparticule selon l'une ou plusieurs des revendications 1 à 11, pour la libération contrôlée de produits agrochimiques.

14. Utilisation de la microparticule selon l'une ou plusieurs des revendications 1 à 11 en tant que composition pharmaceutique.

15. Utilisation de la microparticule selon l'une ou plusieurs des revendications 1 à 11, en tant que composition agrochimique.
